# EUROPEAN PATENT SPECIFICATION

(11) **EP 3 527 986 B1**
(45) Date of publication and mention of the grant of the patent: **03.08.2022**
(21) Application number: 19153680.4
(22) Date of filing: 25.01.2019
(51) Int. Cl.: G01N 33/543, G01N 33/574, A61M 1/36

(54) **METHOD FOR CAPTURING SPECIFIC CELLS**
VERFAHREN ZUR ERFASSUNG SPEZIFISCHER ZELLEN
PROCÉDÉ DE CAPTURE DE CELLULES SPÉCIFIQUES

(30) Priority: 14.02.2018 JP 2018024091
(43) Date of publication of application: 21.08.2019
(73) Proprietor: Sumitomo Rubber Industries, Ltd., Kobe-shi, Hyogo-ken 651-0072 (JP); Yamagata University, Yamagata-shi, Yamagata 990-8560 (JP); Osaka University, Suita-shi, Osaka 565-0871 (JP)
(72) Inventor: MINAGAWA, Yasuhisa, Kobe-shi Hyogo-ken 651-0072 (JP); TANAKA, Masaru, Yonezawa-shi Yamagata 992-8510 (JP); HOSHIBA, Takashi, Yonezawa-shi Yamagata 992-8510 (JP); EMURA, Haruka, Yonezawa-shi Yamagata 992-8510 (JP); MORI, Masaki, Suita-shi Osaka 565-0871 (JP); YAMAMOTO, Hirofumi, Suita-shi Osaka 565-0871 (JP); HARAGUCHI, Naotsugu, Suita-shi Osaka 565-0871 (JP)
(74) Representative: Manitz Finsterwald Patent- und Rechtsanwaltspartnerschaft mbB

(56) References cited:
- EP-A1- 3 244 208
- EP-A1- 3 301 443
- EP-A1- 3 301 444
- WO-A2-2006/108087
- JP-A- H0 210 160
- HE WEI ET AL: "Quantitation of circulating tumor cells in blood samples from ovarian and prostate cancer patients using tumor-specific fluorescent ligands", INTERNATIONAL JOURNAL OF CANCER, JOHN WILEY & SONS, INC, US, vol. 123, no. 8, 15 October 2008 (2008-10-15), pages 1968-1973, XP009112545, ISSN: 0020-7136, DOI: 10.1002/IJC.23717
- ANONYMOUS: "Ficoll-Paque Plus, Intended Use, Instructions 71-7167-00AG", GE HEALTHCARE, February 2007 (2007-02), pages 1-18,

## Description

### TECHNICAL FIELD

The present invention relates to a method for capturing specific cells (namely cancer cells present in blood) from blood, and a method for analysis of specific cells.

### BACKGROUND ART

When cancer cells are formed, they are known to appear in due course in blood or biological fluid. Such cancer cells appearing in blood are called "circulating tumor cells (CTCs)". Thus, it can be expected that the circulating tumor cells may be analyzed, e.g. to evaluate the cancer-treating effect, predict prognosis life expectancy, predict the effect of anticancer drugs before administration, or examine treatment methods based on genetic analysis of cancer cells.

However, a problem exists in that since the number of circulating tumor cells is very small (several to hundreds of cells/1 mL of blood), such cancer cells are difficult to capture.

For example, the CellSearch System is known as a technique for capturing circulating tumor cells. This technique, which involves an antigen-antibody reaction (capture by EpCAM antibody), can only capture cancer cells expressing EpCAM, and the type of cancer cells that can be captured is limited.

EP 3 301 444 A1 is prior art under Art. 54(3) EPC and discloses a method in which at first a solution of poly (2-methoxyethyl acrylate) is injected into a PMMA plate to prepare an analysis device including a multi-well plate with a hydrophilic polymer layer formed thereon.

JP H02 10160 A discloses a method, in which in a first step a polyethyleneimine is coated onto a glass surface of a test tube. Thereafter, the test tube is filled with a dispersion of erythrocytes in physiological saline solution before centrifugation is performed.

EP 3 244 208 A1 discloses centrifuging sampled blood or a biological fluid and capturing specific cells therefrom onto a hydrophilic polymer layer.

Wei He et al. describe in "Quantitation of circulating tumor cells in blood samples from ovarian and prostate cancer patients using tumor-specific fluorescent ligands", INTERNATIONAL JOUNNAL OF CANCER, JOHN WILEY & SONS, INC, US, vol. 123, no. 8, 15 October 2008; DOI: 10.1002/IJC.23717 that highest recovery efficiencies were observed for so called Ficoll and RosetteStep-Ficoll protocols, comprising a step of centrifugation at 1000 G for 20 minutes was performed.

### CITATION LIST

### PATENT LITERATURE

Patent Literature 1: JP 2005-523981 T

### SUMMARY OF INVENTION

### TECHNICAL PROBLEM

The present invention aims to solve the problem and provide a method for capturing specific cells (e.g. many types of cancer cells, including cancer cells not expressing EpCAM), and a method for analysis of specific cells involving the method.

### SOLUTION TO PROBLEM

The present invention relates to a method for capturing specific cells present in blood, the method including: centrifuging sampled blood at a centrifugal force of 1000 to 3000 G; and capturing specific cells therefrom onto a hydrophilic polymer layer, wherein the specific cells are cancer cells.

In the method for capturing specific cells, the sampled blood is preferably diluted before the centrifugation.

Preferably, a buffer solution or a liquid medium is used for the dilution.

The hydrophilic polymer layer is preferably formed of at least one hydrophilic polymer selected from the group consisting of poly(meth)acryloylmorpholine and polymers represented by the following formula (I): 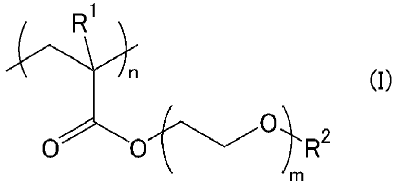 wherein R¹ represents a hydrogen atom or a methyl group, R² represents an alkyl group, m represents 1 to 5, and n represents the number of repetitions.

The hydrophilic polymer layer is preferably formed of a copolymer of at least one hydrophilic monomer selected from the group consisting of (meth)acryloylmorpholine and compounds represented by the following formula (I-1): 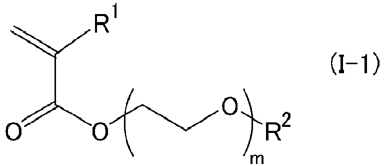 wherein R¹, R², and m are as defined above, with an additional monomer.

The hydrophilic polymer layer preferably has a thickness of 10 to 500 nm.

Another aspect of the present invention relates to a method for analysis of specific cells, including capturing specific cells from blood by the method for capturing specific cells, and analyzing the specific cells.

### ADVANTAGEOUS EFFECTS OF INVENTION

The method for capturing specific cells present in blood according to the present invention includes centrifuging sampled blood at a centrifugal force of 1000 to 3000 G, and capturing specific cells therefrom onto a hydrophilic polymer layer. Such a method can effectively capture specific cells (e.g. many types of cancer cells, including cancer cells not expressing EpCAM). In particular, centrifugation at a centrifugal force as high as 1000 to 3000 G provides improved separation of specific cells from red and white blood cells and the like. Thus, it is possible to sufficiently capture specific cells such as cancer cells from blood and further to reduce adhesion or attachment of blood cells including red and white blood cells and platelets, thereby selectively capturing the specific cells.

### BRIEF DESCRIPTION OF DRAWINGS

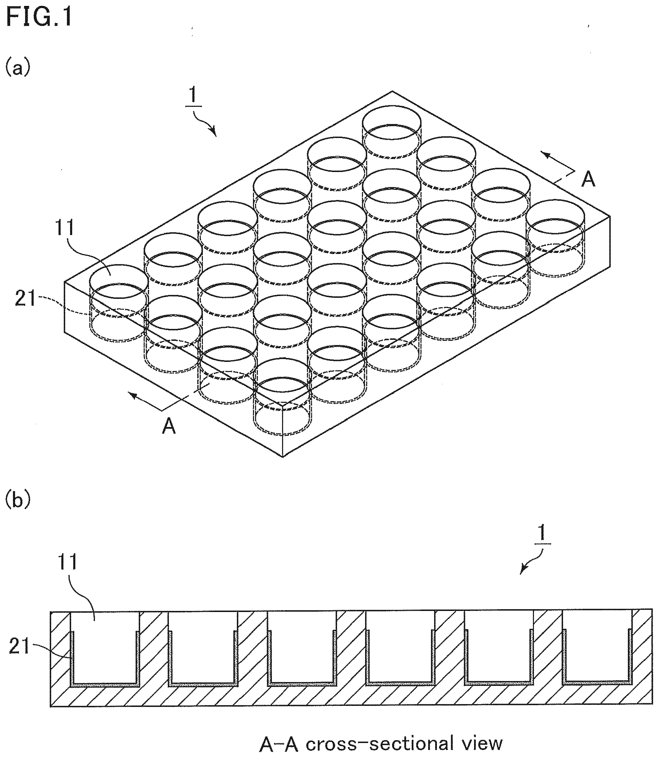

Fig. 1 shows exemplary schematic views of a multi-well plate having wells with a hydrophilic polymer layer formed thereon.

### DESCRIPTION OF EMBODIMENTS

The method for capturing specific cells present in blood or biological fluid of the present invention includes centrifuging sampled blood at a predetermined centrifugal force, and capturing specific cells therefrom onto a hydrophilic polymer layer.

In particular, according to the present method, blood sampled from, e.g. the body may be first centrifuged at a predetermined centrifugal force to prepare a sample having levels of red and white blood cells and the like lower than the sampled blood. Then, the sample may be contacted with a hydrophilic polymer layer to capture specific cells such as cancer cells from the sample. Thus, as the cell adhesion-inhibiting effect of blood cells and the like is reduced, specific cells exhibit their inherent ability to adhere to hydrophilic polymers. Therefore, the present method provides greatly improved capture of specific cells such as cancer cells and reduced capture of blood cells and the like, thereby being effective in selectively capturing the specific cells such as cancer cells to an extent that could never be achieved when high levels of blood cells are present.

More specifically, sampled blood may be previously centrifuged at a predetermined centrifugal force to separate (remove) platelets, red and white blood cells, and the like from the blood, thereby preparing a sample containing them at reduced levels. Then, the sample may be contacted with a hydrophilic polymer layer to selectively capture specific cells. Thus, the tumor cells and the like in the blood can be effectively captured onto the hydrophilic polymer layer. Then, it can be expected that by counting the number of captured tumor cells and the like, one can determine the number of tumor cells and the like in the blood, e.g. in order to evaluate the cancer-treating effect. Moreover, the captured tumor cells and the like may be cultured and then used to determine the effect of drugs such as anticancer drugs. This allows us to determine the effect of drugs such as anticancer drugs ex vivo before administration, and also helps to screen drugs such as anticancer drugs.

Examples of specific cells that may be used in the method for capturing specific cells include cancer cells (any cancer cells, including cancer cells not expressing EpCAM). Examples of the cancer cells include circulating tumor cells (CTCs).

In the method for capturing specific cells, the sampled blood or biological fluid is subjected to centrifugation at a centrifugal force of 1000 to 3000 G (×g). A centrifugal force of 1000 G or higher can provide improved separation of red and white blood cells and the like and reduction in the loss of specific cells such as cancer cells (the loss due to the specific cells being incorporated into the fraction of red blood cells and the like), thereby being effective in selectively capturing specific cells. Also, a centrifugal force of 3000 G or lower can result in reduced stress on specific cells, thereby maintaining their original nature. The centrifugal force is preferably 1300 to 2800 G, more preferably 1500 to 2500 G.

The duration and temperature of the centrifugation may be appropriately selected, e.g. in view of the ability to separate red blood cells and the like. For example, the centrifugation may be performed for 1 to 120 minutes, preferably 1 to 60 minutes, at 2 to 40°C, preferably 3 to 30°C. The centrifugation process may be carried out by known techniques, such as using a known centrifugal separator.

In the centrifugation process, the sampled blood may be centrifuged, followed by removing the supernatant containing platelets to prepare a sample having a platelet level lower than the sampled blood. Moreover, the sampled blood may be centrifuged, followed by separating an intermediate mononuclear cell layer to separate and remove red and white blood cells, thereby preparing a sample with an increased level of specific cells such as cancer cells.

The method for capturing specific cells may include, prior to the centrifugation, an additional treatment to reduce protein levels in the blood. The additional treatment to reduce protein levels in the blood may be carried out, for example, by diluting the sampled blood. The dilution may be performed using a buffer solution such as a phosphate buffered saline (PBS) having the same pH as human blood (about 7.4) or a liquid medium such as Dulbecco's modified eagle's medium (DMEM).

Specifically, it may be carried out by diluting the sampled blood with a buffer solution, or adding the sampled blood to a liquid medium for dilution, to obtain protein levels lower than the sampled blood.

In the method for capturing specific cells, the centrifugation process, optionally preceded by the additional treatment, is followed by capturing specific cells onto a hydrophilic polymer layer.

The hydrophilic polymer layer (the layer formed of a hydrophilic polymer) may be formed on a certain substrate. Examples of the substrate include acrylic resins (polyacrylic resins) such as polymethyl acrylate, polymethyl methacrylate, polyacrylic acid, and polymethacrylic acid; cycloolefin resins (polycycloolefins); carbonate resins (polycarbonates); styrene resins (polystyrenes); polyester resins such as polyethylene terephthalate (PET); polydimethylsiloxanes; and glass such as soda-lime glass and borosilicate glass.

The hydrophilic polymer layer (the layer formed of a hydrophilic polymer) preferably has a thickness of 10 to 500 nm, more preferably 30 to 400 nm, still more preferably 50 to 350 nm. When the thickness is adjusted within the range indicated above, selective capture of cancer cells and low adsorption of other proteins and cells can be well achieved.

The hydrophilic polymer may be appropriately selected from polymers having hydrophilicity. For example, it may be a homopolymer or copolymer of one or two or more hydrophilic monomers, or a copolymer of one or two or more hydrophilic monomers with an additional monomer. Examples of such homopolymers and copolymers include polyacrylic acid, polyacrylic acid esters, polymethacrylic acid, polymethacrylic acid esters, polyacryloylmorpholine, polymethacryloylmorpholine, polyacrylamide, and polymethacrylamide.

The hydrophilic monomers may be any monomer containing a hydrophilic group. Examples of the hydrophilic group include known hydrophilic groups such as an amide group, a sulfuric acid group, a sulfonic acid group, a carboxylic acid group, a hydroxyl group, an amino group, and an oxyethylene group.

Specific examples of the hydrophilic monomers include (meth)acrylic acid, (meth)acrylic acid esters (e.g. alkoxyalkyl (meth)acrylates such as methoxyethyl (meth)acrylate, and hydroxyalkyl (meth)acrylates such as hydroxyethyl (meth)acrylate), (meth)acrylamide, and (meth)acrylamide derivatives containing cyclic groups (e.g., (meth)acryloylmorpholine). Preferred among these are (meth)acrylic acid, (meth)acrylic acid esters, alkoxyalkyl (meth)acrylates, and (meth)acryloylmorpholine, with alkoxyalkyl (meth)acrylates being more preferred, with 2-methoxyethyl acrylate being particularly preferred.

The additional monomer may be appropriately selected as long as it does not inhibit the effects of the hydrophilic polymer. Examples include aromatic monomers such as styrene, vinyl acetate, and N-isopropylacrylamide which can impart temperature responsiveness.

In particular, the hydrophilic polymer is preferably at least one selected from the group consisting of poly(meth)acryloylmorpholine and polymers represented by the following formula (I): 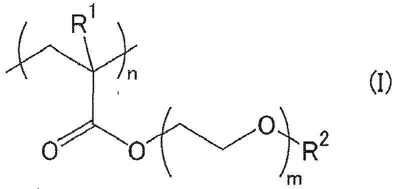 wherein R¹ represents a hydrogen atom or a methyl group, R² represents an alkyl group, m represents 1 to 5, and n represents the number of repetitions.

The alkyl group represented by R² preferably has 1 to 10 carbon atoms, more preferably 1 to 5 carbon atoms. In particular, R² is particularly preferably a methyl group or an ethyl group. The symbol m is preferably 1 to 3. The symbol n (number of repeating units) is preferably 15 to 1500, more preferably 40 to 1200.

Alternatively, the hydrophilic polymer may also suitably be a copolymer of at least one hydrophilic monomer selected from the group consisting of (meth) acryloylmorpholine and compounds represented by the formula (I-1) below with an additional monomer. 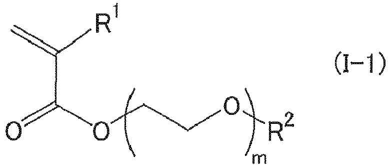 In formula (I-1), R¹, R², and m are as defined above.

The surface of the hydrophilic polymer layer preferably at least partially (partially or entirely) has a contact angle with water of 25 to 75°, more preferably 35 to 75°, still more preferably 35 to 70°. When the hydrophilic polymer layer has such a predetermined contact angle with water, the effects of the present invention can be well achieved.

The hydrophilic polymer layer may be formed by dissolving or dispersing a hydrophilic polymer in any solvent to prepare a hydrophilic polymer solution or dispersion, and entirely or partially coating the surface of a substrate with the hydrophilic polymer solution or dispersion by a known method, such as (1) by injecting the hydrophilic polymer solution or dispersion into the substrate surface (the recess of the substrate) and retaining and drying it for a predetermined time, or (2) by applying (spraying) the hydrophilic polymer solution or dispersion to the substrate surface and retaining and drying it for a predetermined time. Thus, a substrate provided with a polymer layer formed of a hydrophilic polymer can be prepared. Then, the substrate provided with a hydrophilic polymer layer may be combined with other components as needed, to prepare an apparatus capable of analyzing specific cells.

The solvent, injection method, application (spraying) method, and other conditions may be conventionally known materials or methods.

The retention/drying time in the method (1) or (2) may be selected appropriately according to the size of the substrate, the type of liquid introduced, and other factors . The retention time is preferably five minutes to ten hours, more preferably ten minutes to five hours, still more preferably 15 minutes to two hours. The drying is preferably performed at room temperature (about 23°C) to 80°C, more preferably at room temperature to 50°C. Moreover, the drying may be carried out under reduced pressure. Furthermore, the hydrophilic polymer solution or dispersion may be retained for a certain period of time, optionally followed by discharging the excess solution or dispersion before drying.

The solvent may be any solvent that can dissolve the hydrophilic polymer and may be selected appropriately according to the hydrophilic polymer used. Examples include water, organic solvents, and solvent mixtures thereof. Examples of the organic solvents include alcohols such as methanol, ethanol, n-propanol, i-propanol, and methoxypropanol, ketones such as acetone and methyl ethyl ketone, tetrahydrofuran, acetonitrile, ethyl acetate, and toluene.

In the method for capturing specific cells, the sample (sample having lower blood cell levels) prepared by centrifuging blood may be contacted with the substrate provided with a hydrophilic polymer layer to capture specific cells. Contacting the sample with the hydrophilic polymer layer may be carried out by any method capable of this contact, such as by injecting or applying (spraying) the sample.

By contacting the sample with the hydrophilic polymer layer, the specific cells present in the sample can be captured onto the hydrophilic polymer layer while reducing adsorption of blood cells and the like. Thus, the specific cells may be selectively captured onto the hydrophilic polymer layer, for example, by retaining the contacted sample for a predetermined time and then washing it. Then, it can be expected that by counting the number of captured specific cells, one can determine the number of specific cells in the sampled blood, e.g. in order to evaluate the cancer-treating effect.

The method for capturing specific cells may be performed using, for example, a device that includes a substrate such as a multi-well plate or a chamber slide, optionally with additional components. Fig. 1 illustrates an exemplary multi-well plate 1.

The multi-well plate 1 in Fig. 1 is a device intended to capture specific cells in which wells 11 are arranged in so-called matrix form. The multi-well plate 1 has multiple wells 11 having a circular opening. The wells 11 are recesses into which a sample may be injected that is prepared by centrifuging sampled blood at a predetermined centrifugal force to reduce the levels of blood cells and the like. Specific cells can be effectively captured when the injected sample is subjected to analysis as compared to when the sampled blood is directly subjected to analysis. Thus, it is possible to confirm the presence or absence of specific cells in blood, count the number of specific cells, culture the specific cells, determine the effect of drugs, and screen the drugs.

Although Fig. 1 illustrates a 24-well plate having 24 wells 11 arranged in 4 rows by 6 columns as an example, it is sufficient for the multi-well plate 1 to have at least two wells 11, and any number of wells 11 may be provided. Examples other than the 24-well plate include general multi-well plates in which the number of wells 11 is 6, 96, 384, etc.

Each well 11 is a non-through hole which is opened at the surface of the multi-well plate 1. A sample prepared by centrifuging blood may be injected into the wells 11 through the respective openings. If the presence of specific cells is confirmed, a culture fluid for culturing the specific cells may also be injected.

The diameter R of the opening and the depth D of each well 11 are not particularly critical, and may be those of a conventional multi-well plate 1. Although in Fig. 1, the inner side surface of each well 11 is substantially vertical to the opposite faces of the multi-well plate 1, the inner side surface of the wells 11 may be inclined to taper from the opening to the bottom. Alternatively, the inner side surface may be inclined to flare out from the opening to the bottom.

Though the wells 11 in Fig. 1 are circularly opened, the openings of the wells 11 may be of any shape such as quadrangle.

The multi-well plate 1 may suitably be one in which the multiple wells 11 are separable. When multiple wells are provided, they may be separated into wells for counting the number of specific cells and for culturing the specific cells. For example, the presence or absence of specific cells may first be confirmed in the wells for counting, and if the presence is confirmed, the specific cells may then be cultured in the wells for culturing and then used to determine the effect of drugs. In a suitable chamber slide, the number of chambers is at least one but not more than ten.

In the multi-well plate 1 or chamber slide, the wells 11 preferably have a hydrophilic polymer layer formed at least partially on the inner surface thereof. In the example shown in Fig. 1, a hydrophilic polymer layer 21 is formed on the bottom surface and a part of the side surface of the wells.

Once a sample prepared by centrifuging blood is introduced into the wells 11, the specific cells present in the sample can be captured onto the hydrophilic polymer layer 21 while reducing adsorption of blood cells and the like. Thus, the specific cells may be selectively captured onto the hydrophilic polymer layer 21 by retaining the introduced sample for a predetermined time and then washing it.

The method for analysis of specific cells according to the present invention includes capturing specific cells being cancer cells from blood by the method described above, and analyzing the specific cells. This method can capture specific cells (e.g. many types of cancer cells, including cancer cells not expressing EpCAM). Moreover, this method can sufficiently capture specific cells from blood while reducing adhesion or attachment of other proteins and cells, thereby selectively capturing the specific cells.

In the method for analysis of specific cells, the hydrophilic polymer layer may suitably be contacted with blood from which blood cells and the like have been removed. This can further enhance selective capture of specific cells such as cancer cells.

### EXAMPLES

The present invention is specifically described with reference to, but not limited to, examples below.

### (Device Example 1)

Using azobisisobutyronitrile (AIBN), 2-methoxyethyl acrylate was thermally polymerized at 80°C for six hours to produce poly(2-methoxyethyl acrylate) (molecular weight: Mn = about 15,000, Mw = about 50,000). Then, a 1.0% by mass solution of the poly (2-methoxyethyl acrylate) in methanol was prepared.

The poly(2-methoxyethyl acrylate) solution (1% by mass) was injected into the wells of a polystyrene 24-well plate and left for 30 minutes at room temperature. Thereafter, the solution was partly drawn using a pipette, followed by drying to prepare a medical analysis device.

### (Device Example 2)

A medical analysis device was prepared as in Device Example 1, except that the concentration of the poly(2-methoxyethyl acrylate) solution was changed to 2.5% by mass.

### (Device Example 3)

A medical analysis device was prepared as in Device Example 1, except that the concentration of the poly(2-methoxyethyl acrylate) solution was changed to 5.0% by mass.

### (Device Example 4)

A medical analysis device was prepared as in Device Example 1, except that a borosilicate glass chamber slide was used, and the concentration of the poly(2-methoxyethyl acrylate) solution was changed to 0.3% by mass.

### (Device Example 5)

A medical analysis device was prepared as in Device Example 4, except that the concentration of the poly(2-methoxyethyl acrylate) solution was changed to 0.5% by mass.

### (Device Example 6)

A medical analysis device was prepared as in Device Example 1, except that no poly(2-methoxyethyl acrylate) solution was injected and no poly(2-methoxyethyl acrylate) layer was formed.

### [Thickness of hydrophilic polymer layer (coating layer)]

The thickness of the hydrophilic polymer layer of the medical analysis devices was determined by measuring (photographing) a cross section of the hydrophilic polymer layer using a TEM at an accelerating voltage of 15 kV and a magnification of 1000 times.

### [Contact angle with water]

A volume of 2 µL of distilled water was dropped onto the surface of the hydrophilic polymer layer of each medical analysis device. Thirty seconds later, the contact angle was measured by the θ/2 method at room temperature.

### [Analysis of whole blood spiked with cancer cells]

Stained human colon adenocarcinoma (HT-29) cells were suspended in whole blood to a concentration of 100 cells per mL of blood to prepare spiked blood. The spiked blood was diluted with an equal volume of a liquid medium to prepare a spiked blood dilution. Next, to a 15 ml centrifuge tube were added a solution for isolation (Lymphoprep, density = 1.077 ± 0.001 g/mL) and then the spiked blood dilution, followed by centrifugation under conditions indicated in Table 1. Then, the mononuclear cell layer was separated. To the separated mononuclear cell layer was added a phosphate buffer (PBS) solution, followed by centrifugation again to enrich the mononuclear cell layer. After the centrifugation, the aggregates at the lowermost layer were suspended in a liquid medium containing 10% fetal bovine serum (FBS) in a volume equal to the initial whole blood volume. A 1 ml portion of the suspension was injected into each well or chamber and left at 37°C for one hour to cause adhesion. Then, non-adhered cells were washed away with a PBS solution. Thereafter, the number of adhered cancer cells was counted using a fluorescence microscope.

**[Table 1]**

| | Device Exemple 1 | | Device Exemple 2 | | Device Exemple 3 | | Device Exemple 4 | | Device Exemple 5 | | Device Exemple 6 | |
|---|---|---|---|---|---|---|---|---|---|---|---|---|
| | Example 1 | Comparative Example 1 | Example 2 | Comparative Example 2 | Example 3 | Comparative Example 3 | Example 4 | Comparative Example 4 | Example 5 | Comparative Example 5 | Comparative Example 6 | Comparative Example 7 |
| Centrifugation conditions at RT (about 23°C ): Centrifugal force Duration | 2000G 5 min | 800G 20 min | 2000G 5 min | 800G 20 min | 2000G 5 min | 800G 20 min | 2000G 5 min | 800G 20 min | 2000G 5 min | 800G 20 min | 2000G 5 min | 800G 20 min |
| Thickness of hydrophilic plymer layer (coating layer) (nm) | 39 | 39 | 86 | 86 | 280 | 280 | 108 | 108 | 450 | 450 | 0 | 0 |
| Contact angle with water (° ) | 43 | 43 | 50 | 50 | 60 | 60 | 52 | 52 | 65 | 65 | 85 | 85 |
| Analysis of whole blood spiked with cancer cells (number of cells) | 71 | 41 | 80 | 46 | 85 | 55 | 66 | 45 | 75 | 48 | 48 | 47 |

When a sample prepared by centrifuging blood at a centrifugal force of 1000 to 3000 G was contacted with a hydrophilic polymer layer (coating layer), specific cells such as cancer cells were selectively captured, and the number of adhered specific cells was increased.

### REFERENCE SIGNS LIST

- 1: multi-well plate
- 11: well
- 21: hydrophilic polymer layer

## Claims

1. A method for capturing specific cells present in blood, the method comprising:
centrifuging sampled blood at a centrifugal force of 1000 to 3000 G; and
capturing specific cells therefrom onto a hydrophilic polymer layer,
wherein the specific cells are cancer cells.

2. The method for capturing specific cells according to claim 1,
wherein the sampled blood is diluted before the centrifugation.

3. The method for capturing specific cells according to claim 2,
wherein a buffer solution or a liquid medium is used for the dilution.

4. The method for capturing specific cells according to any one of claims 1 to 3,
wherein the hydrophilic polymer layer is formed of at least one hydrophilic polymer selected from the group consisting of poly(meth)acryloylmorpholine and polymers represented by the following formula (I): 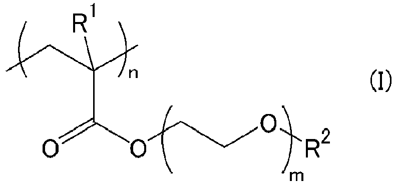 wherein R¹ represents a hydrogen atom or a methyl group, R² represents an alkyl group, m represents 1 to 5, and n represents the number of repetitions.

5. The method for capturing specific cells according to any one of claims 1 to 3,
wherein the hydrophilic polymer layer is formed of a copolymer of at least one hydrophilic monomer selected from the group consisting of (meth)acryloylmorpholine and compounds represented by the following formula (I-1): 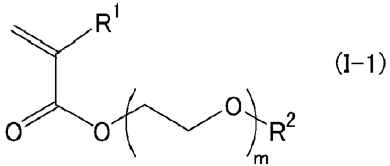 wherein R¹ represents a hydrogen atom or a methyl group, R² represents an alkyl group, and m represents 1 to 5, with an additional monomer.

6. The method for capturing specific cells according to any one of claims 1 to 5,
wherein the hydrophilic polymer layer has a thickness of 10 to 500 nm.

7. A method for analysis of specific cells, comprising capturing specific cells from blood by the method for capturing specific cells according to any one of claims 1 to 6, and analyzing the specific cells.

## Patentansprüche

1. Verfahren zum Auffangen spezifischer Zellen, die in Blut vorhanden sind, wobei das Verfahren umfasst:
Zentrifugieren einer Blutprobe bei einer Zentrifugalkraft von 1000 bis 3000 G; und
Auffangen spezifischer Zellen aus derselben auf einer hydrophilen Polymerschicht,
wobei die spezifischen Zellen Krebszellen sind.

2. Verfahren zum Auffangen spezifischer Zellen nach Anspruch 1,
wobei die Blutprobe vor der Zentrifugation verdünnt wird.

3. Verfahren zum Auffangen spezifischer Zellen nach Anspruch 2,
wobei eine Pufferlösung oder ein flüssiges Medium für die Verdünnung verwendet wird.

4. Verfahren zum Auffangen spezifischer Zellen nach einem der Ansprüche 1 bis 3,
wobei die hydrophile Polymerschicht gebildet ist aus mindestens einem hydrophilen Polymer, welches ausgewählt ist aus der Gruppe bestehend aus Poly(meth)acryloylmorpholin und durch die folgende Formel (I) dargestellten Polymeren: 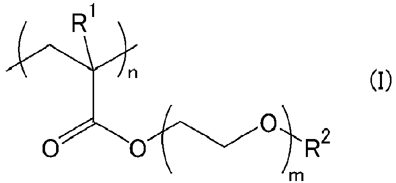 wobei R¹ ein Wasserstoffatom oder eine Methylgruppe darstellt, R² eine Alkylgruppe darstellt, m 1 bis 5 darstellt, und n die Anzahl an Wiederholungen darstellt.

5. Verfahren zum Auffangen spezifischer Zellen nach einem der Ansprüche 1 bis 3,
wobei die hydrophile Polymerschicht gebildet ist aus einem Copolymer mindestens eines hydrophilen Monomers, welches ausgewählt ist aus der Gruppe bestehend aus (Meth)acryloylmorpholin und durch die folgende Formel (I-1) dargestellten Verbindungen: 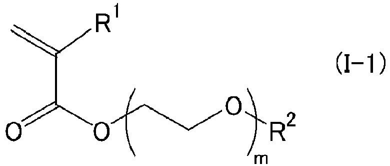 wobei R¹ ein Wasserstoffatom oder eine Methylgruppe darstellt, R² eine Alkylgruppe darstellt, und m 1 bis 5 darstellt, mit einem zusätzlichen Monomer.

6. Verfahren zum Auffangen spezifischer Zellen nach einem der Ansprüche 1 bis 5,
wobei die hydrophile Polymerschicht eine Dicke von 10 bis 500 nm aufweist.

7. Verfahren zur Analyse spezifischer Zellen, umfassend das Auffangen spezifischer Zellen aus Blut durch das Verfahren zum Auffangen spezifischer Zellen nach einem der Ansprüche 1 bis 6 und das Analysieren der spezifischen Zellen.

## Revendications

1. Procédé de capture de cellules spécifiques présentes dans le sang, le procédé comprenant les étapes consistant à :
centrifuger le sang échantillonné à une force de centrifuge de 1 000 à 3 000 G ; et
capturer les cellules spécifiques de celui-ci sur une couche de polymère hydrophile,
les cellules spécifiques étant des cellules cancéreuses.

2. Procédé de capture de cellules spécifiques selon la revendication 1,
dans lequel le sang échantillonné est dilué avant la centrifugation.

3. Procédé de capture de cellules spécifiques selon la revendication 2,
dans lequel une solution tampon ou un milieu liquide est utilisé pour la dilution.

4. Procédé de capture de cellules spécifiques selon l'une quelconque des revendications 1 à 3,
dans lequel la couche de polymère hydrophile est formée d'au moins un polymère hydrophile choisi dans le groupe constitué par la poly(méth)acryloylmorpholine et les polymères représentés par la formule (I) suivante : 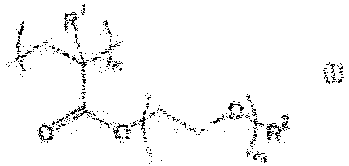 dans laquelle R¹ représente un atome d'hydrogène ou un groupe méthyle, R² représente un groupe alkyle, m représente 1 à 5, et n représente le nombre de répétitions.

5. Procédé de capture de cellules spécifiques selon l'une quelconque des revendications 1 à 3,
dans lequel la couche de polymère hydrophile est formée d'au moins un copolymère d'au moins un monomère hydrophile choisi dans le groupe constitué par la (méth)acryloylmorpholine et les composés représentés par la formule (I-1) suivante : 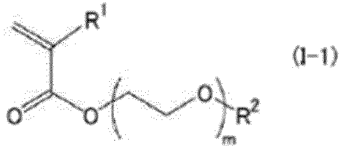 dans laquelle R¹ représente un atome d'hydrogène ou un groupe méthyle, R² représente un groupe alkyle, et m représente 1 à 5, avec un monomère supplémentaire.

6. Procédé de capture de cellules spécifiques selon l'une quelconque des revendications 1 à 5,
dans lequel la couche de polymère hydrophile a une épaisseur de 10 à 500 nm.

7. Procédé d'analyse de cellules spécifiques, comprenant la capture de cellules spécifiques du sang par le procédé de capture de cellules spécifiques selon l'une quelconque des revendications 1 à 6 et l'analyse des cellules spécifiques.
